Europäisches Patentamt

European Patent Office

Office européen des brevets

(11) Publication number: **0 438 595 A1**

# EUROPEAN PATENT APPLICATION
## published in accordance with Art. 158(3) EPC

(21) Application number: 90910193.3

(22) Date of filing: 10.07.90

(86) International application number:
PCT/JP90/00884

(87) International publication number:
WO 91/00766 (24.01.91 91/03)

(51) Int. Cl.5: **B01D 29/05, G01N 33/48**

(30) Priority: 12.07.89 JP 179642/89
20.12.89 JP 330399/89

(43) Date of publication of application:
31.07.91 Bulletin 91/31

(84) Designated Contracting States:
AT BE CH DE DK ES FR GB IT LI LU NL SE

(71) Applicant: SIBASAKI, Michiro
6-7-19, Osawa, Mitaka-shi
Tokyo 181(JP)

(72) Inventor: SIBASAKI, Michiro
6-7-19, Osawa, Mitaka-shi
Tokyo 181(JP)

(74) Representative: LLOYD, Patrick Alexander
Desmond et al
Reddie & Grose 16 Theobalds Road
London WC1X 8PL(GB)

(54) **JIG AND DEVICE FOR FILTRATION.**

(57) A jig used to filter and catch specimens such as cells and provided with a filter having numerous pores and to a filtering device used to hangle this jig. The jig is provided with a water-retentive piece (3) and the jig body (4) with a communicating hole (7) and a communicating hole (7'). The water-reten- tive piece (3) facilitates wet fixation of the specimen and the two holes (7), (7') increase the operatability. The filtering jig is provided with a pressing part (47) for preventing the filter (6) from being clogged. Fix- ation of the filtering jig to the filtering device enables quick and easy catch and staining of the specimen.

Fig.1

Fig.1'

## TECHNICAL FIELD

This invention related to a filtration jig which is used to collect material such as cells on a filter, and a filtration device which operates the filtration Jig.

Technical Background of the Invention

Making a preparation is necessary when material such as cells is examined. In the past, the preparation is usually made by smearing material on a slide. Since this process is done by hand, it required a considerable amount of time and a highly trained technic.

Concerning these inconveniences, the inventor of the present invention had invented a filter holder and applied it (Japanese Patent Application No. 61-250188) prior to the present invention. This holder is arranged to hold a membrane filter which has a countless number of minuteness pores, through which solution is filtered collecting the material thereon. The holder comprises a filtration passage and a check valve (non-return valve) therein, and the solution is drained through the passage by a vacuume pump, collecting the material on the filter.

The problem of this prior art is that when a wet fixation is to be made with this holder, an extra work is required. As it is known, there are two types of fixations: one is a dry fixation which keeps the material dry; and the other is a wet fixation which keeps the material wet. When a wet fixation is to be made with the prior holder, water has to be provided continuously on a filter after it is removed from the holder to prevent the material from being dryed. This is not an easy work.

Another problem of this prior art is that a passage is formed either through the bottom surface or the side surface of the body, and the passage is arranged to attach either a vacuume device or for a hand operation. When a lot of preparations are to be made, the mechanical device should be applied because a lot of materials can be collected in a shorter period of time, and when only a few preparations are to be made, it is easier to collect the material by hands using as such as a syringe. However, the prior art comprises only one passage which passes through either the bottom surface or the side surface, and the passage is arranged for either for the device or the hand operation, so that it is an inconvenient holder. Therefore, a holder that is applicable for both the device and the hand operation is strongly desired.

The first object of the present invention therefore is to provide a holder which makes the work of wet fixation easier, and which can be applied both for the mechanical vacuume device and the hand operation.

Another problem of the prior art is that a filter easily clogs, especially when the filter is flat, because pores of such filter are So small that a diameter thereof is between a few $\mu$m to several tens $\mu$m. When collecting cells in such as urine and cerebrospinal fluid which includes a lot of protein, the filter easily clogs by the protein.

The second object of the present Invention, therefore, is to provide a filtration jig which prevents a filter from being clogged.

And, the third object of the present invention is to provide a filtration device which operates the filtration jig according to the present invention.

Description of the Invention

To achieve the first object, the holder according to the present invention comprises an upper body 1, a filter body 2 comprising a frame 5 and a filter 6 fixed thereto, the filter 6 having a countless number of pores therethrough, a water holder 3 which shapes like a thin plate and is absorbent, and a main body 4 comprising a passage 7 vertically passes through the middle of the body 4 and another passage 7' connected between the passage 7 and the side surface of the body 4. The main body 4 comprises from the bottom of its top portion a water holder 3, a filter body 2 and an upper body 1, capable of removing.

It is necessary to install a check valve to prevent the back flow of the liquid in the passages 7, 7'. A common stopper can be applied in the passages 7, 7' as the device. However, to further improve the sealing ability, the device in the passage 7 is provided with a stopper 9 and a spring 10. According to this device, while the stopper 9 is pushed downward by an elastic force of the spring 10, the passage is closed by the stopper 9, and when the stopper 9 is pushed upward, the passage 7 opens. Further, it is arranged such that when the stopper 9 is pushed upward, it pushes the water holder 3 and disconnects the holder 3 from the main body 4.

To prevent the back flow in the passage 7', either a ball 9' and a spring 10 or a stopper 9 and a spring 10 can be comprised, and by pushing the ball 9' or the stopper 9 by an elastic force of the spring 10, the passaged 7' can be closed, and by pushing the ball 9' or the stopper 9 against the elastic force of the spring 10, the passage 7' can be opened.

A pair of grips 12 can be secured to the side surfaces of the main body 4 to hold the upper body 1 by its elastic force. Projections 13 can be secured on the top surface of the main body 4 to settle the position of the filter body 2.

Further, a set-up concave 15 can be formed on the top surface of the main body 4 to hold the water holder 3 therein, and a plural number of support projections 16 can be secured in the passage 7 to support the water holder 3 from underneath. An O-ring 17, the bottom surface thereof being connected airtightly to the top surface of the filter body 2, can be positioned in the bottom portion of the upper body 1. A packing 18 is placed in the concave 15 of the main body 4, holding the water holder 3 airtightly and the top surface thereof is being connected airtightly to the bottom surface of the filter body 2. A groove 14 own be formed on the top surface of the main body to remove the filter body 2.

To accomplish the second object, the filtration jig according to the present invention comprises a upper body 1, filter body 2, a water holder 3 and a main body 4. The upper body 1 shapes like a tube and solution including the material is applied therein. The filter body 2 consists of a frame 5 and a filter 6 with numberless pores secured thereto. The water holder 3 is absorbent and shapes like a thin plate. The main body 4 comprises a passage 7 vertically runs through in the middle thereof, and it holds on its top surface the water holder 3, the filter body 2 connected to the water holder 3 and the upper body 1, all of them being capable of removing. The water holder 3 is arranged to revolve in one direction or both direrctions at regular intervals by a rotary means 46.

To prevent the filter 6 from being clogged, a remover 47 is secured to the top surface of the water holder 3, which provides an effective water pressure to the bottom surface of the filter 6, or which directly contacts the bottom surface of the filter 6, removing any substances clogged in the pores of the filter 6.

The filtration device, which achieves the third object of the present invention, comprises a fixed stand 19 which holds a filtration jig according to the present invention, a nozzle 20 whose nozzle exit 23 is connected to the passage 7 opened at the bottom of the filtration jig, an elevating plate 21 which moves upward to connect the nozzle exit 23 to the passage 7 and moves downward to disconnect the nozzle exit 23 from the passage 7, and a suction means 22 which filtrates solution in the filtration jig.

A roller pump can be used as a suction means 22, and a means that supplies a fixing agent can be comprised.

Functions of the filtration jig and device according to the present invention are described below.

First, the function of the filtration is described. A water holder 3 is held on a main body 4, then a filter body 2 is placed on the water holder 3. By creating a set-up concave on the main body 4 and placing a packing 18 therein, the water holder 3 can be held air-tightly. Also, by arranging a projection 13 on the main body 4, the filter body 2 can be placed on a right position.

Then, an upper body 1 is installed an the main body 4. By securing a pair of grips 12 to the main body 4 which holds the upper body 1, and a O-ring at the bottom of the upper body 1 which touches the top surface of the filter body 2, the filtration jig can be held airtightly without a leak of solution.

After completing an installation of the Filtration jig according to the present invention, solution which contains material is supplied in the upper body 1. Then, the solution is drained, collecting the material on the filter 6. According to the present invention, the main body 4 comprisies not only a passage 7, the opening thereof is positioned in the bottom surface of the main body 4, but also another passage 7', the opening thereof is located in the side surface of the body 4, so that a hand operation with a syringe can be performed easily, since the passage 7' in the side surface can be seen by eyes.

When a solution is vacuumed through the passage 7' or the other passage 7', the stopper 9 or the ball 9' in the passages 7, 7' is pushed back against the elastic force of the spring 10 by a nozzle 23 of a vacuume device making an opening therein. By this vacuume process, the solution is drained through the countless number of pores and the material is collected on the filter 6.

When a dry fixation of the material filtered on the filter 6 is required, the filter body 2 is removed from the main body 4 with a means such as a pincette. In this case, by arranging a removing concave on the top surface of the main body 4 and inserting the pincette therein, the filter body 2 can be easily removed.

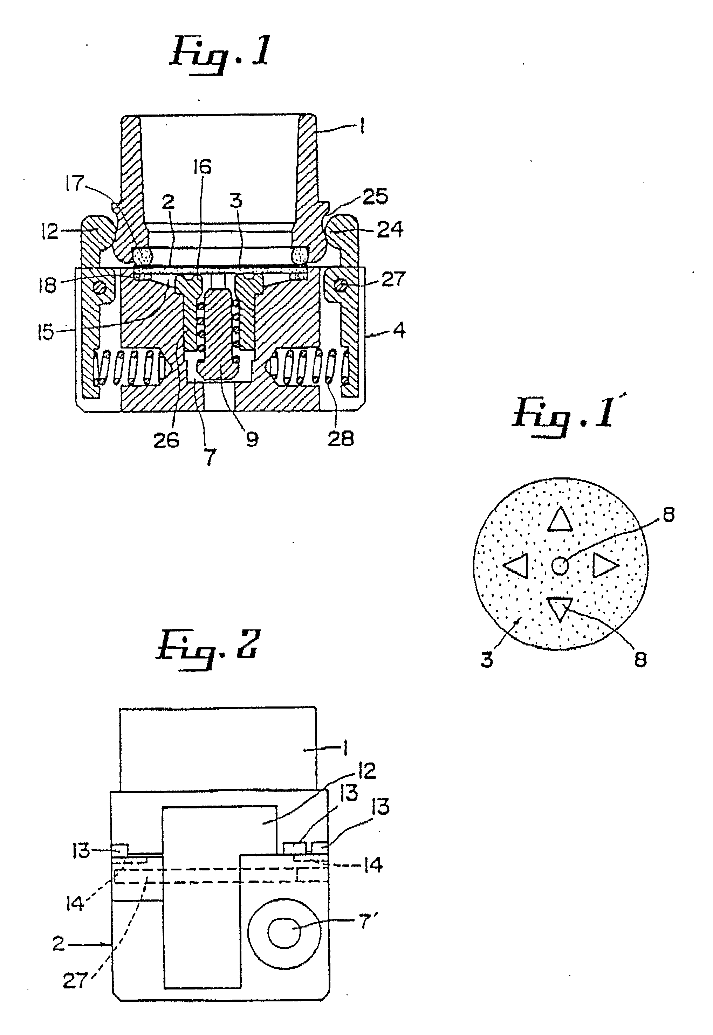

When a wet fixation of the material is needed, the stopper 9 in the passage 7 should be pushed upward from the bottom by a means such as a thin bar, so that the top portion of the stopper 9 pushes the water holder 3, removing the water holder 3 and the filter body 2 together from the main body 4(See Fig. 1). Even a set of the water holder 3 and the filter body 2 can be left as it is, moisture of the water holder 3 is continuously provided to the material, so that the material does not dry for a long time and the dry fixation can be prepared.

After the filtration, the oultivation or the dyeing of the material, the filter body 2 is placed on top of a semitransparent slide (Japanese Patent Application No. 63-75701, applied by the same inventor). In this case, by an effect of light spreading of the semitransparent slide, pores of the filter 6 are not observed, so that the material is clealy examined. With this method, it is not necessary to rely on the smearing process which is not an efficient way to make a preparation and which requires a highly trained technic, so that anyone can make a good preparation easily.

Effects of a filtration jig which accomplishes the second object of the present invention will be explained below. Solution including material is supplied in the upper body 1, then it is sucked through the passage 7, filtering the material on the filter 6. In case, the pores of the filter 6 are clogged by this filtration, a rotary means 46 is connected to the water holder 3, and the rotates the water holder 3. The bottom surface of the filter 6 and the top surface of the filter are connected each other, so that when the water holder 3 rotates, the bottom surface of the filter 6 is scrubbed by the water holder 3, removing substances such as protein clogged in the pores of the filter 6.

By securing a remover 47 on top of the water holder 3, a hydraulic pressure caused by a rotation of the water holder 3 or by a brushing effect of the remover 47, the substances clogged in the pored of the filter 6 are easily removed.

Effects of a filtration device which achieves the third object of the present invention will be explained below. First, the filtration according to the present invention is secured to the fixed stand 19. Then, the elevating plate 21 is lifted upward, pushing the stopper 9 of the jig upward by the nozzle exit 23 and opening the passage 7. After solution including material is supplied in the upper body 1, the suction means 22 is activated to suck and drain the solution through the passage 7 and to filter the material on the filter 6. After completing the filtration, cultivation or dyeing of the material, another filtration jig can be secured to the device for a same process.

Brief Description of the Drawings

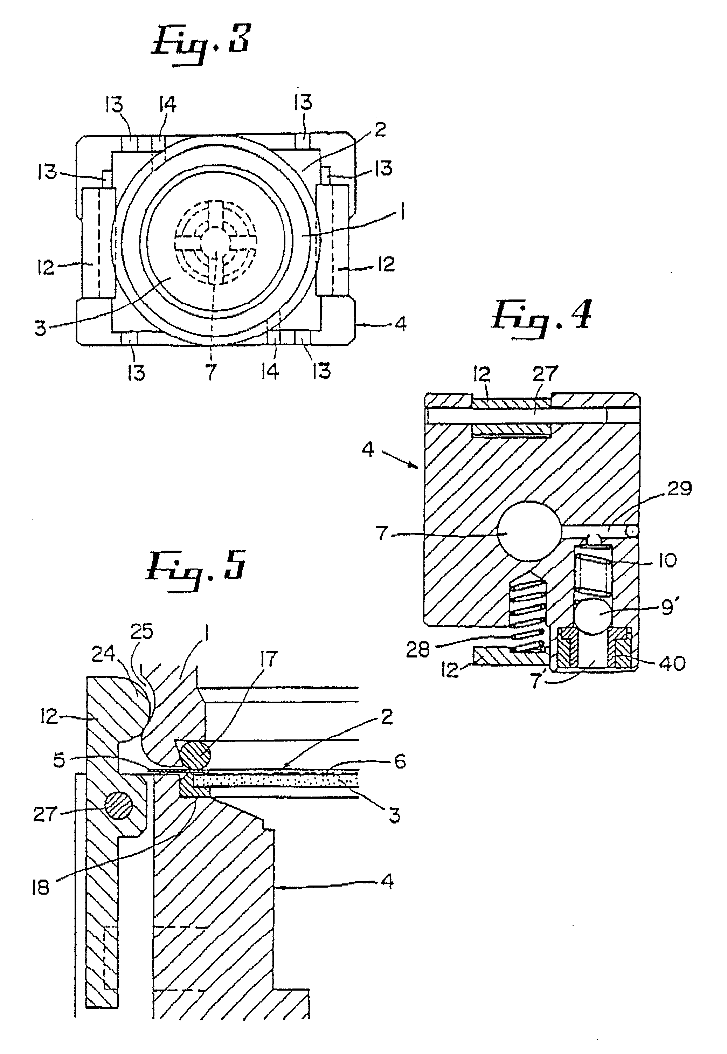

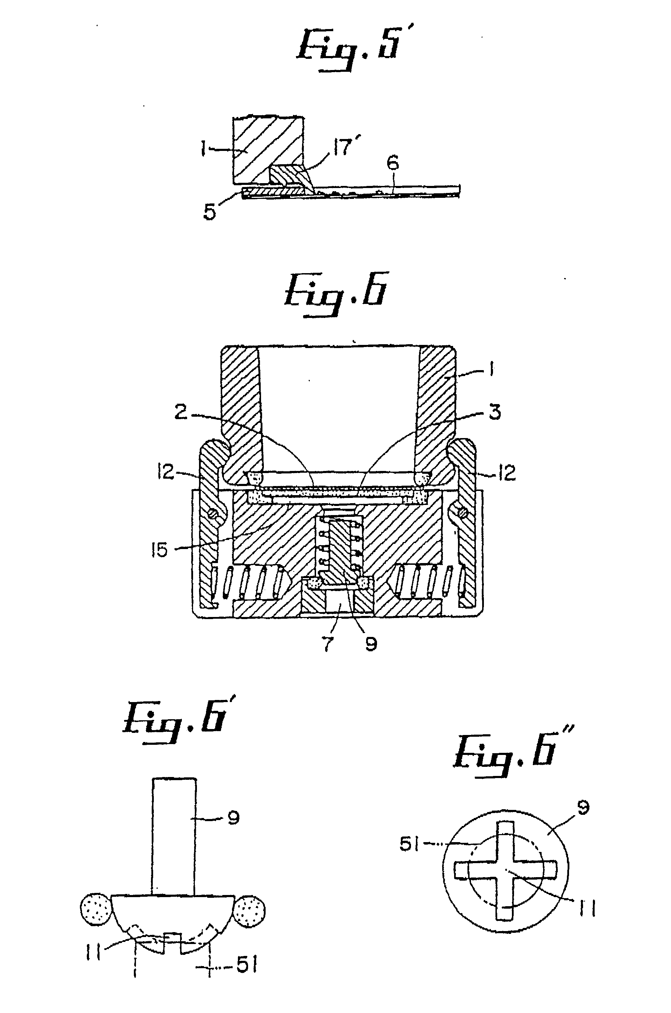

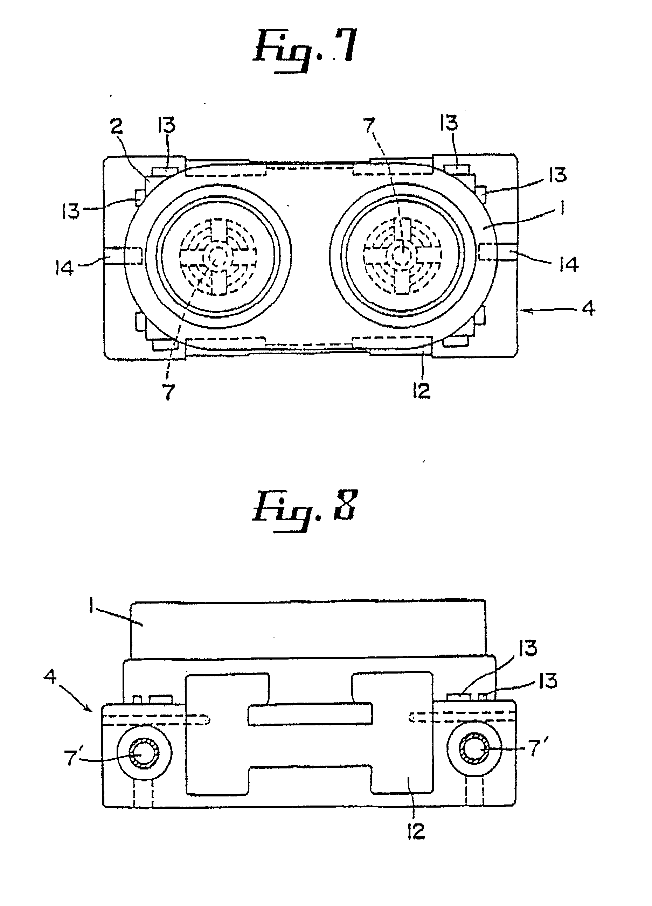

Fig. 1 is an elevational view in section of a preferred embodiment of the filtration jig according to the present invention, Fig. 2 is a aide elevational view of the embodiment shown in Fig. 1, Fig. 3 is a top view of the embodiment shown in Fig. 1, Fig. 4 is a transverse sectional view OF the embodiment shown in Fig. 1 , Fig. 5 is a partially enlarged view of the embodiment shown in Fig. 1, Fig. 6 is an elevational view in section of another preferred embodiment of the filtration jig according to the present invention, Fig. 7 is a top view of another preferred embodiment of the filtration jig according to the present invention, Fig. 8 is a side elevational view of the embodiment shown in Fig. 7.

Fig. 1' is a top view of a preferred embodiment of the water holder 3, Fig. 5' is an elevational view in section of a preferred embodiment of a packing installed to the bottom portion of the upper body, Fig. 6' is an elevational view of a preferred embodiment of the stopper, Fig. 6" is a bottom plan view of the embodiment shown in Fig. 6'.

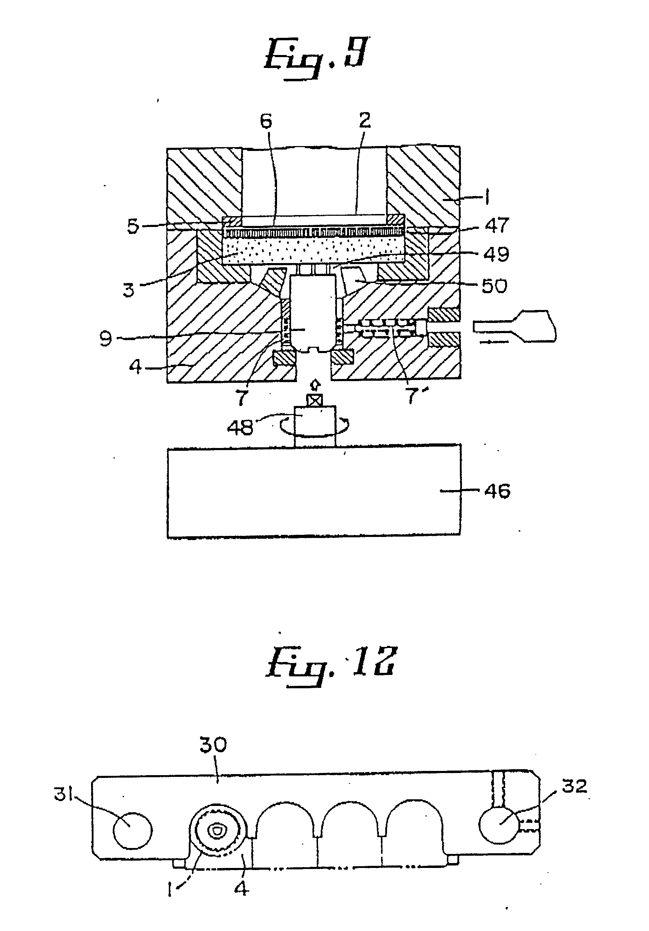

Fig. 9 is an elevational view in section of a preferred embodiment of the filtaration jig according to the present invention which prevents clogging of the filter.

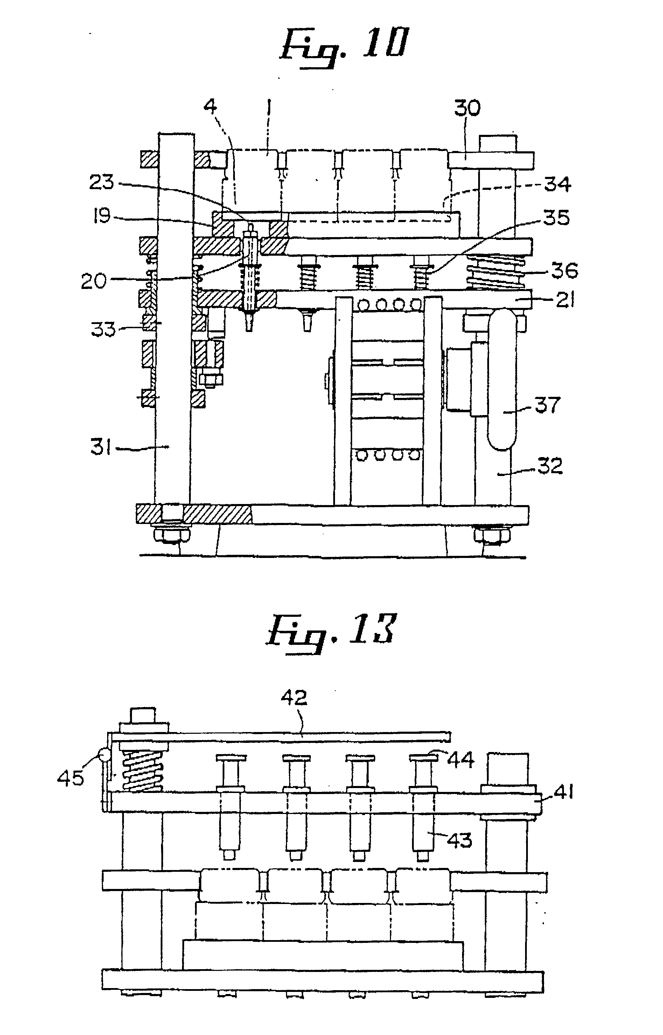

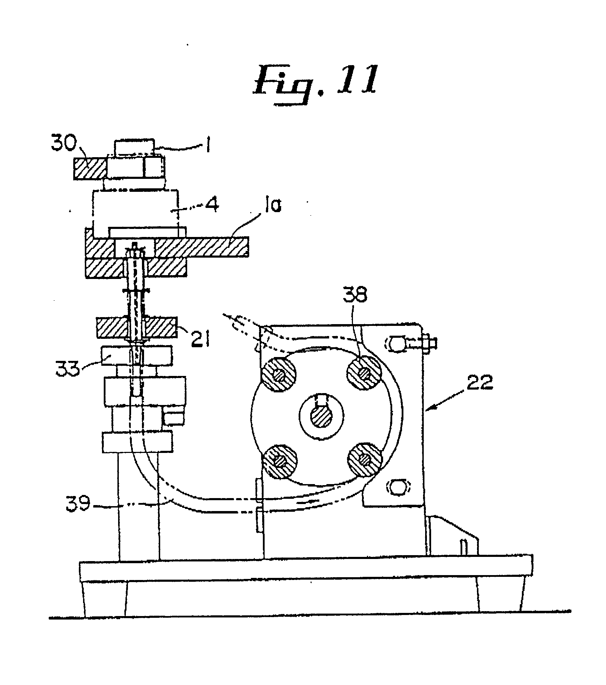

Fig. 10 is an eleavational view partially in section of a preferred embodiment of the filtration device according to the present invention, Fig. 11 is a side elevational view of the embodiment shown in Fig. 10, Fig. 12 is a top view in partial of the embodiment shown in Fig. 10. Fig. 13 is an elevational view of a preferred embodiment of a means for supplying a fixing agent.

Preferred Embodiment of the Present Invention

Fig. 1 to 5 show a preferred embodiment of the filtration jig which achieves the first object of the present invention.

The main body 4 is a plastic made and is shaped like rectangular, and the passage 7 runs vertically the center thereof. A support tube 26 is secured in the passage 7, which comprises a number of support projections 16, and the spring 10 and the stopper 9 are installed in the support tube 26. The set-up concave 15 arranged on the top portion of the main body 4 declines toward the opening of the passage 7, so that solution is naturally led to the passage 7.

Another passage 7' opened to the side wall of the main body 4 is connected to the other passage 7 via a connect passage 29. In the passage 7', a steel ball stopper 9', a spring 10 and a seal 40 are secured, and the stopper 9' is caused to press the seal 40 by an elastic force of the spring 10, closing an opening of the passage 7. The stopper 9' is pushed by a nozzle 20 of a sucking means, thereby opening the closed passage 7 to filtrate the solution. In stead of the steel made ball stopper 9', a pin-shaped stopper 9 which is installed in the other passage 7 can be applied.

The main body 4 comprises six projections 13 for setting the filter body 2, four of them are to guide a longtudinal direction and the others are to guide a bilateral direction of the filter body 2.

The main body 4 comprises a pair of grips 12, the upper portions thereof are supported by pine 27 capable of rotating and the lower portions thereof are connected to a spring 28. The grips 12 are caused to hold the water holder 3 by an elastic Force of the spring 28. Each of the grips comprises a projection 24. and the upper body 1 comprises a concave 25 around its lower portion. The projection 24 pushes the concave 25 downward, holding the water holder 3 tightly.

The upper body 1 is a hard plastic made and is shaped like a cylinder. The upper body 1 comprises a groove around its inner lower portion, and a soft plastic made O-ring 17 is positioned in the groove The bottom surface of the O-ring 17 is airtightly connected to the frame 5 of the filter body 2, thereby preventing a leak of solution in the upper body 1 (see Fig. 5). Arranging the upper body 1 transparent or semitransparent and drawing a scale on the wall thereof, the amount of the solution supplied in the upper body 1 can be scaled with eyes.

The filter body 2 comprises a frame 5 which is a rectangle and is 0.1 ˜ 0.2 mm thick, and a membrane filter 6 which covers the frame 5. The membrane filter 6 is several tens thick and comprises a countless number of pores which is several $\mu$m in diameter. Solution containing material is filtrated by the membrane filter 6 and the material is collected on the filter 6.

The water holder 3, secured to the concave 15 Of the main body 4, is shaped like a circle and is made of material which preserves a lot of liquid.

Fig. 6 shows another preferred embodiment of the filtration jig according to the present invention. This jig excludes a support tube 26 and a support projectin 16, and the bottom surface of the concave 15 is arranged flat, making this jig simpler.

Besides the embodiments described above, the jig according to the present invention can be arranged to comprise a number of main bodies 4 and upper bodies 1. In such a case, each of them can be connected either capable or incapable of separation. Figs. 7 and 8 show an embodiment comprising two main bodies 4 connected incapable of separation, and comprising a filter body 2 composed of a long frame 5 and a filter.

A preferred embodiment of the filtration jig which pursue the second object of the present invention is shown in Fig. 9. This embodiment shows a basic construction of the invention. According to this embodiment, the water holder 3 revolves by a rotation of the stopper 9, which is connected to a bar 48 and rotates by a force of the rotary means 46. A number of pins 49 are secured on top of the stopper 9, and which are sticked to the water holder 3. The power source of the rotary means 46 can be an electric motor, a magnet and even a manpower. The water holder 3 comprises a remover 47 thereon, which is arranged like a brush and which removes substances clogged in the pores of the filter 6. The remover 47 can be comprised by an electrostatic deposition of thin substances.

The remover 47 can be made other than the brush. For example, a groove or a projection can be formed on top of the water holder 3. By rotating the water holder 3 and producing water pressure by an effect of the groove or the projection, the substances clogged in the pores can be removed by the water pressure.

According to this preferred embodiment, the main body 4 comprises a number of projections 50. The projections 50 apply water pressure to the bottom of the water holder 3 or they even scrub the bottom thereof, removing the clogged substances.

Figs. 10, 11 and 12 show a preferred embodiment of the filtaration device which achieves the third object of the present invention.

The fixed stand 19 is secured still between a first support 31 and a second support 32. The stand 19 comprises an install concave 34 on which four filtration jigs can be placed. The fixed stand 19 can be used as a work desk by enlarging its size. It can be arranged transparent, so that things thereunder can be seen therethrough, thereby making the work easier.

Right above the fixed stand 19, a hold plate 30 is secured between the first support 31 and the second support 32, which holds the upper bodies 1.

The elevating plate 21 is secured between the first support 31 and the second support 32 capable of elevation. Springs 36 are installed between the first support 31 and the second support 32, pushing the elevation stand 21 downward by an elastic force thereof. A control plate 33 is secured to the elevating plate 21. By a rotation of the control plates 33, the elevating plate 21 is caused to elevate against an elastic force of the springs 36. Four nozzles 20 are secured to the elevating plate 21. The nozzles 20 elevate with an elevation of the elevating plate 21, and the nozzle exits 23 push the stoppers 9 in the passages 7 of the main body 4, thereby opening the passages 7.

The elevating plate 21 can be elevated by a mechanical means instead of the control plate 33 which is operated by hands.

After a completion of the filtration, the control plates 33 are rotated in the reversed direction, lowering the elevating plate 21 by a elastic force of the springs 36 and removing the nozzle exits 23 from the passages 7. Each nozzle 20 is secured by a spring 35 to secure the nozzle exit 23 and the passage 7 tightly.

The nozzles 20 are connected to the suction means, a roller pump in this case. Plastic tubes 39 connected to the nozzles 20 are stripped off by rollers 38 which revolve by a handle 37, thereby filtering the solution in the jig. Instead of the roller pump, other means such as an air pump can be applied,

A dyeing method such as Papanicolaou is a wet fixation, so that the material collected on the filter 6 must not be dried or half-dried, and that a fixing agent must be applied immediately. According to the jig shown in Figs. 7 and 8, a fixing agent is supposed to apply to each filter by hand. Such work can be accomplished quickly at once by securing the jig to a means for supplying a fixing agent shown in Fig. 13. According to this means, a number of Syringes 43 are comprised to a rotary plate 41 on lines of radial directions from the center thereof, and each syringe 43 is positioned right above each jig. A lever 45 is pulled down, thereby pushing syringe heads 44 by a press bar 42 and supplying a fixing agent in the upper bodies 1. Wiht this means, the fixing agent can be supplied to a number of jigs at once, so that it is a good advantage for a wet fixation which requiers a quick operation. The rotary plate 41 can be shaped like a circle, and by rotating the plate 41, a set of syringes lined an a radial direction on the plate 41 can be positioned right above the jigs.

Industrial Utilization of the Invention

The filtration jig directed to resolve the first object comprises a stopper in the passage, and by pushing the stopper upward, the water holder and the filter body can be removed together at once. It is a good advantage for preparing a wet fixation.

The filtration jig according to the present invention, the passages are formed through the bottom surface and the side surface of the main body, so that a mechanical device can be applied to the passage of the bottom surface and a hand operation with a syringe can be performed through the passage of the side surface. Therefore, either or both passages can be used depending on how many preparations are to be made. This makes the work much eisier.

The filtration jig aiming the second object of the present invention makes a water holder capable of revolving, so that clogged substances in the pores of a filter can be removed and the filter is prevented from being clogged. This is a remarkable advantage especially when filtering abdominal drospy and fluid in the thoracic cavity, which includes considerable protein.

The filtration device according to the present invention is invented to easily handle the filtration jig, so that the jig is well operated with the device, further improving the efficiency in making preparations.

**Claims**

1. A filtration jig which comprises:
   an upper body, wherein the solution including material is being supplied;
   a water holder, being absorbent and shaped like a thin plate;
   a main body, comprising a passage vertically passing through in the center thereof, and another passage connected to said passage, an opening thereof being formed in the side surface of said body, said main body holding its top portion said water holder, a filter body and said upper body, capable of removing.

2. A filtration jig claimed in claim 1, wherein each of said passages being provided with a means for preventing a back flow of liquid.

3. A filtration jig claimed in claim 2, wherein said means for preventing a back flow comprising a stopper and a spring, said stopper being pushed downward by an elastic force of said spring, closing said passage, said stopper being pushed upward by said spring, opening laid passage, said water holder being removed from said main body by being pushed by said stopper.

4. A filtration jig calimed in claim 2 or 3, wherein said means for preventing a back flow comprising a ball and a spring or a stopper and a spring, said ball or said stopper being pushed downward by an elastic force of said spring, closing said passage, said ball or said stopper being pushed against an elastic force of said spring, opening said passage.

5. A filtration jig calimed in claim 1, 2, 3 or 4, wherein said main body being provided at its opposing side walls with a pair of grips for holding said upper body.

6. A filtration jig claimed in claim 1, 2, 3, 4 or 5, wherein said main body being provided on its top surface with projections for positioning a filter body.

7. A filtration jig claimed in claim 1, 2, 3, 4, 5 or 6, wherein a groove for removing said filter body being secured on the top surface of said main body.

8. F filtration jig calimed in claim 1, 2, 3, 4, 5 or 6, wherein said main body comprising a concave on the top surface thereof for receiving said water holder, said main body also comprising support projections in said passage for supporting said water holder from beneath.

9. A filtration jig claimed in claim 1, 2, 3, 4, 5, 6, 7 or 8, wherein said upper body having an O-ring, the bottom surface of said O-ring being connected airtightly to the top surface of said filter body, said main body having a packing in said concave for holding said water holder airtightly, the top surface of said packing being connected air tightly to the bottom surface of said filter body.

10. A filtration jig which comprises:

an upper body, wherein the solution including material is being supplied;

a water holder, being absorbent and shaped like a thin plate;

a main body, comprising a passage vertically passing through in the center thereof, and another passage connected to said passage, an opening thereof being formed in the side surface of said body, said main body holding its top portion said water holder, a filter body and said upper body, capable of removing,

said water holder being revolved in one direction or both directions at regular intervals by a rotary means.

11. A filtration jig claimed in claim 10, wherein said water holder comprising a remover for preventing said filter from being clogged.

12. A filtration device comprising:

a fixed stand for securing a filtration jig capable of removing;

a nozzle, a nozzle exit thereof being capable of connection to a passage opened to the bottom surface of said main body;

an elevating plate, being arranged to move upward and downward securing said nozzle, and to connect said nozzle exit to said passage at the top position, and to remove said nozzle exit from said passage at the bottom position;

a suction means, being arranged to filter solution in said filtration jig, connecting to the bottom of said nozzle.

13. A filtration device claimed in claim 12, wherein said suction means is a roller pump.

14. A filtration device claimed in claim 12 and 13. further comprising a means for supplying a fixing agent, which comprises:

a rotary plate securing a number of syringes in radial directions positioning said syringes right above said filtration jigs;

and a press bar being arranged to press a syringe head of said syringe by a lever.

**_Fig.1_**

**_Fig.1´_**

**_Fig.2_**

**Fig.3**

**Fig.4**

**Fig.5**

Fig. 5'

Fig. 6

Fig. 6'

Fig. 6"

# Fig. 7

# Fig. 8

# Fig. 9

# Fig. 12

# Fig. 10

# Fig. 13

Fig. 11

# INTERNATIONAL SEARCH REPORT

International Application No PCT/JP90/00884

**I. CLASSIFICATION OF SUBJECT MATTER** (if several classification symbols apply, indicate all) [6]

According to International Patent Classification (IPC) or to both National Classification and IPC

Int. Cl$^5$     B01D29/05, G01N33/48

**II. FIELDS SEARCHED**

| Minimum Documentation Searched [7] | |
|---|---|
| Classification System | Classification Symbols |
| IPC | B01D29/00-29/05, B01D27/00, G01N33/48 |

Documentation Searched other than Minimum Documentation
to the Extent that such Documents are Included in the Fields Searched [8]

Jitsuyo Shinan Koho      1926 - 1990
Kokai Jitsuyo Shinan Koho      1971 - 1990

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 64-063011 (Michio Shibazaki), 9 March 1989 (09. 03. 89), Line 5, column 1 to line 1, column 2, lines 3 to 12, column 8 (Family: none) | 1 - 11 |
| A | JP, A, 63-103971 (Yugen Kaisha Japan Menbren), 9 May 1988 (09. 05. 88), Line 5, column 1 to line 13, column 2, lines 10 to 12, column 9 (Family: none) | 12 - 14 |
| A | JP, A, 60-166014 (Tokyo Electric Power Corp. and five others), 29 August 1985 (29. 08. 85), Lines 9 to 12, column 1, lines 16 to 20, column 4 (Family: none) | 10, 11 |
| A | JP, A, 55-165114 (Nippondenso Co., Ltd.), 23 December 1980 (23. 12. 80), Lines 5 to 10, column 1 (Family: none) | 3, 4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure; use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 18, 1990 (18. 09. 90) | October 1, 1990 (01. 10. 90) |
| International Searching Authority | Signature of Authorized Officer |
| Japanese Patent Office | |